# EUROPEAN PATENT APPLICATION

(11) **EP 3 989 123 A1**
(43) Date of publication of application: **27.04.2022**
(21) Application number: 20827035.5
(22) Date of filing: 04.06.2020
(51) Int. Cl.: G06N 3/00

(54) **GENETIC CIRCUIT SIMULATING ARTIFICIAL NEURAL NETWORK AND CONSTRUCTION METHOD THEREFOR**

(30) Priority: 21.06.2019 CN 201910541858
(71) Applicant: CHI, U Seak, Hong Kong (CN)
(72) Inventor: CHI, U Seak, Hong Kong (CN); LO, LOK In, Hong Kong (CN); KUO, Wai Keung, Hong Kong (CN)
(74) Representative: Basck Ltd
(86) International application number: PCT/CN2020/094416
(87) International publication number: WO 2020/253547

(57) **Abstract**

A genetic circuit simulating an artificial neural network, comprising at least one input layer, at least two hidden layers, and at least one output layer. The input layer comprises several input layer nodes, each of the hidden layers comprises several hidden layer nodes, the output layer comprises several output layer nodes, input vectors of each input layer node comprise a promoter and an input gene, each hidden layer node comprises a hidden layer gene, and each hidden layer gene is regulated and controlled by the promoter of the gene of that layer or a gene product of the gene of the previous layer; the output node outputs a genetic circuit result; the regulation and control to the hidden layer gene and the output layer node by the product of the gene of the previous layer conforms with an activation function; the regulation and control to the input layer node by the input promoter conforms with an activation function. A genetic circuit structure is used to simulate an artificial neural network, so that complex operation is simulated at the molecular level, and complex application in the fields of biology, medicine, chemistry, electronics and the like is achieved.

## Description

### TECHNICAL FIELD

The present invention relates to the field of biology, and more particularly, to a gene circuit for simulating an artificial neural network and a construction method therefor.

### BACKGROUND OF THE INVENTION

Artificial neural network (Artificial Neural Network, ie, ANN) has been a hot research topic in the field of artificial intelligence since the 1980s. It abstracts the human brain neural network from the information processing angle to establish simplified models and forms different networks in different connection manners. An Artificial neural network is also commonly referred to as a neural network in the field of engineering and academia. A neural network is a mathematical model composed of a large number of nodes (or referred to as neurons). Each node represents a particular output function, referred to as an activation function. The connection between every two nodes represents a weighting value for the connection signal, which is referred to as a weight, which is equivalent to the memory of the artificial neural network. The output of the network is based on the connection of the networks, the weight value, and the activation function. The network itself is usually an approximation of a certain algorithm or function in nature, and may also be recognised as a logical expression.

The gene circuit is usually composed of regulators and regulated genes, and the regulator comprises a promoter or the like, and the regulated gene is usually a protein-coding gene. The current gene circuit design mainly comprises a basic logic loop design and a detecting/sensing circuit or the like. However, these gene circuit designs do not have the processing speed of a general computer, nor are capable of doing the complex calculation, and therefore, the practical application is limited.

### SUMMARY OF THE INVENTION

The technical problem to be solved by the present invention is to provide a gene circuit for simulating an artificial neural network and a construction method thereof for the above-mentioned defects in the prior art. This allows the simulation of an artificial neural network by using a gene circuit structure, to simulate a complex operation at a molecular level, thereby implementing a complex application in each biological field.

The technical solution mentioned in the present invention solves the technical problem thereof: constructing a gene circuit for simulating an artificial neural network, comprising at least one input layer, at least two hidden layers and at least one output layer, wherein the input layer comprises a plurality of input layer nodes, each hidden layer comprises a plurality of hidden layer nodes, the output layer comprises a plurality of output layer nodes. The input vector of each input layer node is an input gene, each hidden layer node comprises a hidden layer gene, and each hidden layer gene is regulated by a promoter of that layer or a gene product of the previous layer of the gene. The output layer nodes output a gene circuit result; and the hidden layer and the output layer nodes are subjected to the regulation and control of the gene product(s) of the previous layer of gene(s), to meet the activation function.

In the gene circuit for simulating an artificial neural network, mentioned in the present invention, the gene product is a protein or an RNA.

In the gene circuit for simulating an artificial neural network, mentioned in the present invention, the output layer is used for classification analysis or regression analysis.

In the gene circuit for simulating an artificial neural network, mentioned in the present invention, the input layer comprises three input layer nodes, including promoters regulated by ESR 1, PGR, ERBB2, respectively, and the three input genes (Shox2, Rhox11, Lrx3) regulated by its specific promoter. The hidden layer comprises the first hidden layer and the second hidden layer, the first hidden layer comprises six hidden layer genes, and the second hidden layer comprises three hidden layer genes, and these hidden layer genes are comprised of ALX4, OTX1, Hoxc 10, Pknox2, Hoxd13, Dlx1, En2, Prrx2 and Lhx8; and the activation function is a "Sigmoid" function.

In the gene circuit for simulating an artificial neural network, mentioned in the present invention, the result of the gene circuit is a classification of subtype breast cancer: Basal, HER2, and Luminal (ER +).

In the gene circuit for simulating an artificial neural network, mentioned in the present invention, the gene circuit results are the drug PD-L1 for the BASAL subtype breast cancer, the drug Nelipepimult-S for the HER2 subtype breast cancer, and the drug Goserelin for the Luminal (ER +) subtype breast cancer.

In the gene circuit for simulating an artificial neural network, mentioned in the present invention, the input layer comprises three input layer nodes, each input node comprises promoters controlled by copper, lead, cadmium respectively; and three input genes (Fhl -1, Reb -1, Sfp -1) regulated by that specific promoter. The hidden layer comprises the first hidden layer, the second hidden layer, the third hidden layer and the fourth hidden layer. The first hidden layer and the fourth hidden layer both comprise seven hidden layer genes. The second hidden layer and the third hidden layer both comprise four hidden layer genes which the hidden layer genes comprise Alx4, Otx1, Hoxc 10, Pknox 2, Hoxd 13, Dlx1, En2, Prrx2, Lhx8, Gbx2, Msx3, Vsx1, and Barhl1, Hoxd11, Shox2, Rhox11, Lrx3, Hoxd10, Gbx1, Dlx2. The activation function is a "Sigmoid" function or a "ReLU" function, and the output layer comprises a green fluorescent protein.

The technical solution mentioned in the present invention solves the technical problem thereof: constructing a method to allow the design of gene circuit for simulating an artificial neural network, and the method comprises the following steps:

### S1. Analog Training of Artificial Neural Network

S2, Constructing a gene circuit based on the Artificial Neural Network simulation result, wherein the gene circuit comprises at least one input layer, at least two hidden layers and at least one output layer, wherein the input layer comprises a plurality of input layer nodes, each hidden layer comprises a plurality of hidden layer nodes, and the output layer comprises a plurality of output layer nodes. The input vector of each input layer node is a promoter and an input gene, each hidden layer node comprises a hidden layer gene, and each hidden layer gene is regulated by a promoter of that layer or a gene product of the previous layer gene. The output layer nodes output a gene circuit result; and the hidden layer and the output layer nodes are subjected to the regulation and control of the gene product(s) of the previous layer of gene(s), to meet the activation function; based on the gene circuit, selecting the number and type of the input gene and hidden genes, and the number of hidden layers;

S3. The gene circuit is used as an actual expression.

In the construction method of the present invention, the input layer comprises three input layer nodes, comprising a promoter regulated by ESR 1, PGR, ERBB2 respectively, and three input genes (Shox2, Rhox11, Lrx3) regulated by its specific promoter. The hidden layer comprises the first hidden layer and the second hidden layer, the first hidden layer comprises six hidden layer genes, and the second hidden layer comprises three hidden layer genes, which the hidden layer genes comprise AlX4, Otx1, Hoxc10, Pknox2, Hoxd13, Dlx1, En2, Prrx2 and Lhx8; and the activation function is a "Sigmoid" function.

In the construction method of the present invention, the gene circuit result is a classification of the Basal, HER2, and Luminal (ER +) subtype breast cancer; or the gene circuit result is a classification of the drug PD-L1 for the BASAL subtype breast cancer, the drug nelipepimut-S for the HER2 subtype breast cancer, and the drug Goserelin for the Luminal (ER +) subtype breast cancer.

In the construction method of the present invention, the input layer comprises three input layer nodes, and the input genes each comprises a promoter controlled by heavy metal copper, lead, cadmium respectively, and three input genes (Fhl -1, Reb -1, Sfp -1) regulated by that specific promoter, wherein the hidden layer comprises the first hidden layer, the second hidden layer, the third hidden layer and the fourth hidden layer. The first hidden layer and the fourth hidden layer both comprise seven hidden layer genes. The second hidden layer and the third hidden layer both comprise four hidden layer genes. The hidden layer genes comprise ALX4, Otx1, HOCX10, Pknox2, Hoxd13, Dlx1, En2, Prrx2, LHX8, Gbx2, Msx3, Vsx1, Barhl1, Hoxd11 and Shox2. The activation function is a "Sigmoid" function or a "ReLU" function, and the output layer comprises a green fluorescent protein.

According to the gene circuit for simulating an artificial neural network and the construction method thereof, mentioned in the present invention, the gene circuit structure can be adopted to simulate the artificial neural network, so that complex operation can be simulated at the molecular level, and then the complex application in the fields of biology, medicine, chemistry, electronics and the like is realized.

### BRIEF DESCRIPTION OF THE DRAWINGS

The present invention will be further described below with reference to the drawings and embodiments, in which:
Fig. 1 is a schematic diagram of a first embodiment of a gene circuit of a simulated artificial neural network according to the present invention;
Fig. 2 is a schematic diagram of a second embodiment of a gene circuit of a simulated artificial neural network according to the present invention;
Fig. 3 is a schematic diagram of a determination result obtained by the gene circuit shown in Fig. 2;
Fig. 4 is a schematic diagram of a third embodiment of a gene circuit of a simulated artificial neural network according to the present invention;
Fig. 5 is a schematic diagram of a determination result obtained by the gene circuit shown in Fig. 4;
Fig. 6 is a flowchart of a method for constructing a gene circuit of a simulated artificial neural network according to the present invention.

### DETAILED DESCRIPTION OF THE EMBODIMENTS

In order to make the objectives, technical solutions, and advantages of the present invention clearer, the present invention will be further described in detail below with reference to the accompanying drawings and embodiments. It should be understood that the specific embodiments described herein are merely used to explain the present disclosure, and are not intended to limit the present disclosure.

The invention relates to a gene circuit for simulating an artificial neural network. The gene circuit comprises at least one input layer, at least two hidden layers and at least one output layer, wherein the input layer comprises a plurality of input layer nodes, each hidden layer comprises a plurality of hidden layer nodes, the output layer comprises a plurality of output layer nodes. The input vector of each input layer node is a promoter and an input gene, each hidden layer node comprises a hidden layer gene, and each hidden layer gene is controlled by the promoter of that layer or the gene product of the previous layer gene, and the output node outputs a gene circuit result; the hidden layer gene and the output layer node are subjected to regulation and control of the gene product of the previous layer of the gene to meet the activation function, and the input layer node is subjected to regulation and regulation of the input promoter to meet the activation function. According to the gene circuit for simulating the artificial neural network and the construction method thereof, the artificial neural network can be simulated by adopting the gene circuit structure, so that complex operation can be simulated at the molecular level, in order to achieve the complex application in different biological fields.

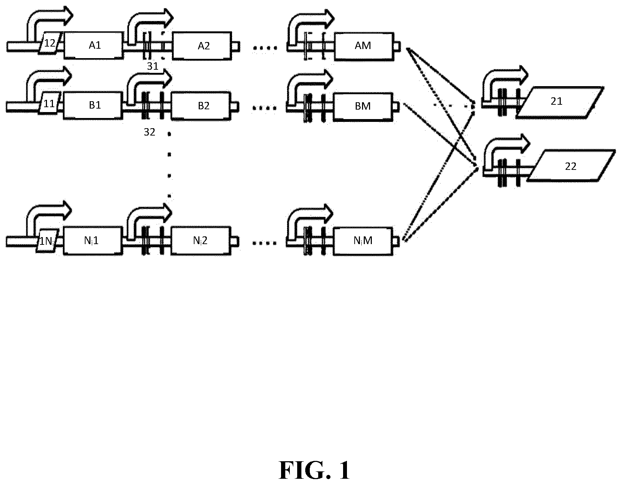

Fig. 1 is a schematic diagram of a first embodiment of a gene circuit of a simulated artificial neural network according to the present selected invention. As shown in Fig. 1, the gene circuit of the artificial neural network includes an input layer, M hidden layers, and an output layer. In the present embodiment, the input layer comprises Ni input nodes (where 1 ≤ i ≤M), those input nodes are 11-1Ni. Each hidden layer comprises Ni hidden layer nodes. The output layer includes two output nodes. In this embodiment, both N and M can be a positive integer greater than 2. It will be appreciated by the expert in this field that the specific values of the Ni and M may be searched according to actual needs, and that searching method may be adjusted by simulating a plurality of groups of artificial neural networks via means of a computer to compare and find out a better solution. The number of hidden layer nodes (Ni) in each hidden layer may be the same or different, and the number of output nodes and the output nodes may also be adjusted according to actual requirements. Here, the present invention is not limited by the specific numerical values thereof.

In this embodiment, the input vector of each input layer node is an input gene, and each hidden layer node includes a hidden layer gene. The input layer node may be controlled by an external Golay molecule, such that the input gene 11-1Ni is used as an input vector. The hidden layer gene A1-Ni1 in the first hidden layer is regulated by the promoter or gene product of the input gene 11-lNi and generates a gene product 31-3Ni, and the gene product 31-3 Ni further regulates the hidden layer gene A2-Ni2 in the second hidden layer and generates a gene product. The regulation of the product of the previous layer of the gene by the hidden layer conforms to the activation function. In this way, the gene product of each layer can be regulated and controlled by the hidden layer gene in the next layer, so that the final output is regulated and controlled, thus the effect of the artificial neural network is achieved. Finally, the output result is formed by the P (P is ≥ 1 and ≤ NM positive integers) group of output genomes.

Optionally, each gene may have a group of independent promoters, RBS (ribosome binding site), and/or terminators. The gene product may be a protein or RNA, for example, a transcription factor, a non-transcribed RNA, or the like. Preferably, in the present invention, at least two input genes are included, and the output result may be a protein or RNA product used for detection (eg, fluorescent protein, fluorescent RNA, or the like) or pharmaceutical purpose (eg, antibody, ligand or the like). In the present invention, the input gene (≤ N group) can be either a DNA sequence (such as a DNA binding site), an RNA or a protein structure. The artificial neural network can be adjusted by computer simulation training, thereby selecting a suitable number of hidden layers and the number of hidden layer nodes in each hidden layer, and an activation function adopted, and selecting an adopted input gene, a promoter, a gene product and a hidden layer gene. In the present invention, for example, a "Sigmoid" function or a "ReLU" function may be selected. In other selected embodiments of the present invention, other activation functions may also be used.

After constructing the gene circuit of the simulated artificial neural network of the present invention, the circuit can be applied to numerous different systems such as animal and plant cells, human cells, bacteria, fungi and cell-free systems for expression. Furthermore, by modifying the input and output circuits, the gene circuit of the artificial neural network according to the present invention may be modified to mimic other artificial neural network gene circuits, such as a recurrent neural network. Optionally, the present invention can combine with the basic logic loop gene circuit design to construct a more accurate Classification or Regression analysis gene circuit.

According to the gene circuit for simulating the artificial neural network, the artificial neural network can be simulated by adopting the gene circuit structure, so that complex operation can be simulated at the molecular level, in order to realize complex applications in biological fields. The gene circuit of the artificial neural network disclosed by the invention enables intracellular or molecular level biological calculation to reach the standard required by current mainstream computer science and is favourable for realizing rapid and accurate medical detection and testing. At the same time, it is also beneficial to realize personalized medical treatment and allow diagnosis and medical treatment at the cellular and molecular levels.

The gene circuit of the simulated artificial neural network according to the present invention is further described below with reference to the embodiments as follows.

### Embodiment 2

Recently, multiple cancers including breast cancer are demonstrated to contain a variety of different cancer cells within the same tumor (referring to "Breast Cancer: Multiple subtypes Within A Tumor?", Trends in Cancer 3.11 (2017): 753-7760, by Yeo Syn Kok and Guan Jun-Lin and "Pan- cancer inference of intra-tumor heterogeneity reveals associations with different forms of genomic instability", Geranium Genetics 14.9 (2018): E1007669, by Raymond Franck, et al.). The following applies the gene circuit of the simulated artificial neural network of the present invention to perform simulation calculation on related cells, and perform breast cancer subtype determination using the output result thereof.

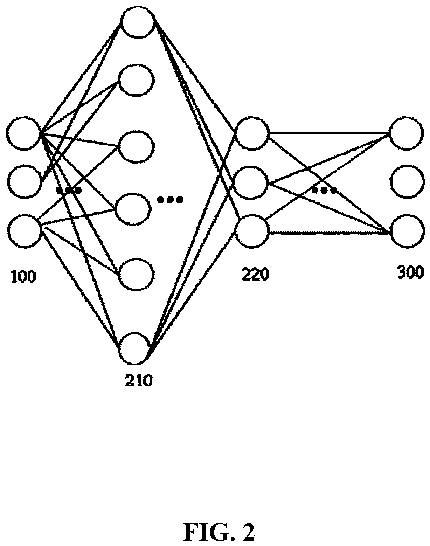

Fig. 2 is a schematic diagram of a second embodiment of a gene circuit of the simulated artificial neural network of the present invention, which can be used for breast cancer subtype determination and/or for different breast cancer subtype drug outputs. The gene circuit of the simulated artificial neural network shown in Fig. 2 comprises an input layer (labelled as 100), a first hidden layer (labelled as 210) and a second hidden layer (labelled as 220), the input layer (labelled as 100) comprises three input layer nodes, the first hidden layer (labelled as 210) comprises six hidden layer nodes, and the second hidden layer (labelled as 220) comprises three hidden layer nodes, and the output layer (labelled as 300) comprises three output nodes. In this embodiment, the activation function employed is a "sigmoid" function.

In the present embodiment, the input vectors input from the input node are respectively ESR 1, PGR and ErbB2 (the promoter is respectively regulated by the three genes, and three promoters respectively regulate the three input genes, such as SHOX2, Rhox11, LRX3), and nine hidden layer genes, such as ALX4, Otx1, HOXC10, Pknox2, Hoxd13, Dlx1, En2, Prrx2 and Lhx8 (referring to "Proteogenomics connects somatic mutations to signalling in breast cancer", Nature, 534.7605 (2016): 55 by Meltins Philippon, et al.) are used as the three hidden layer nodes of the first hidden layer (labelled as 210) and the three hidden layer nodes of the second hidden layer (labelled as 220).In other preferred embodiments of the present invention, other genes may be selected for regulation, such as Gbx2, Msx3, Vsx1, Barhl1, Hoxd11, Shox2, Rhox11, LRX3, Hoxd10, Gbx1 and Dlx2 (referring to "Proteogenomics," Proteogenomics and Connections to Signalling in Breast Cancer. Nature: 534.7605 (2016): 55, by Meltins Philippon, et al.). In the present selected invention, the selected genes match the activation function "sigmoid", and the gene of the next layer is regulated and controlled respectively until the output layer is subjected to Classification analysis for targeted therapy. The present embodiment uses the previously published data (referring to "Proteogenomics connects somatic mutations to signalling in breast cancer". Nature: 534" 7605 (2016): 55, by Merrins Philippon, et al.). The application inputs ESR1, PGR, and ErbB2 expression quantity as an input, and a determination result of the Bayes, HER2, and Luminal (ER +) subtype is used as an output.

In other selected embodiments of the present invention, other numbers of hidden layers or other numbers of genes for each hidden layer may also be selected, for example, three hidden layers may be selected, each hidden layer comprising three hidden layer genes, or two hidden layers may be selected, each hidden layer comprising two hidden layer genes. Table 1 shows the classification accuracy obtained by three options as an example.

| 1^{st} Hidden Layer | 2^{nd} Hidden Layer | 3^{rd} Hidden Layer | Accuracy | Accuracy with random data | P Value |
|---|---|---|---|---|---|
| 3 | 3 | N/A | 65.54 (20.06) | 33.57 (16.84) | 0.00678 |
| 3 | 3 | 3 | 65.54 (20.06) | 42.68 (24.88) | 0.10853 |
| 6 | 3 | N/A | 69.29 (21.79) | 33.57 (16.84) | 0.00165 |

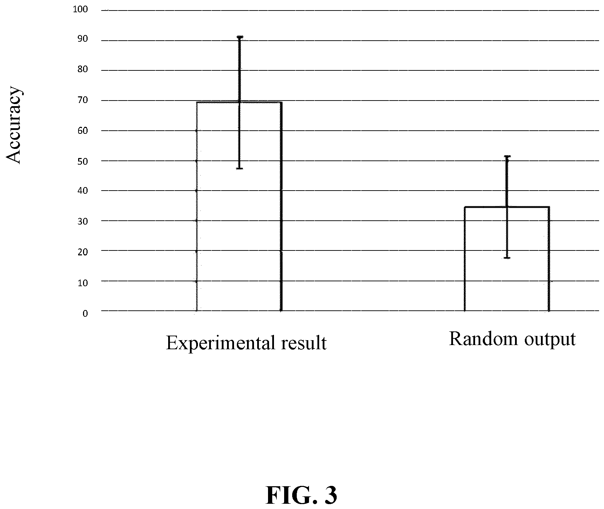

In a further selected embodiment of the present invention, the gene circuit of the simulated artificial neural network shown in Fig. 2 is used to treat different subtypes of cancer as a related treatment, as shown in Fig. 3. In Fig. 3, it shows using the gene circuit of the simulated artificial neural network shown in Fig. 2 can effectively determine the drug carrier cells through the gene circuit of the simulated artificial neural network for different cancer cells (in this embodiment as an example in Basal, HER2 and Luminal (ER +)), so as to make relevant treatment for heterogeneous cells or different subtypes in the same tumor. The result of the gene circuit is that the drug PD-L1 is synthesized for the gene synthesis drug PD-L1 for the BASAL subtype breast cancer, and the drug GellipIPET-S is synthesized for the gene of the HER2 subtype breast cancer, and the drug Goserelin is synthesized for the gene of the Luminal (ER +) subtype breast cancer.

### Embodiment 3

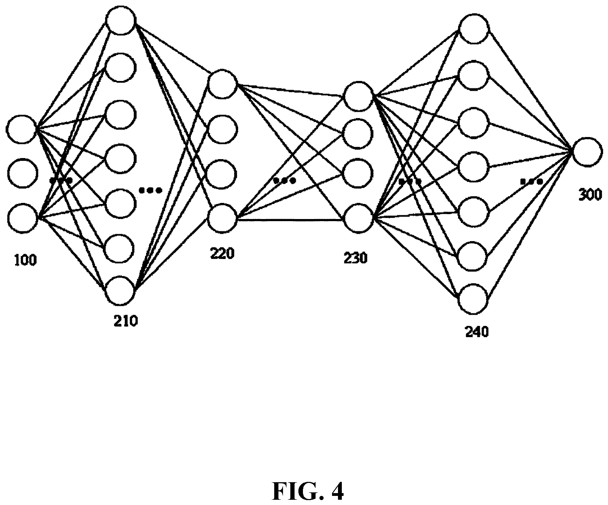

The gene circuit of the artificial neural network of the present invention can be used to perform a Regression analysis to predict the result which saves the number of programs, time and expense required for actual testing. Fig. 4 is a schematic diagram of a third embodiment of a gene circuit of a simulated artificial neural network according to the present invention, which can be used for predicting the toxicity of heavy metals (copper, lead, cadmium) in water, and outputting a toxicity prediction result by using a fluorescent protein as an output layer.

The gene circuit of the simulated artificial neural network shown in Fig. 4 includes an input layer (labelled as 100), the first hidden layer (labelled as 210), the second hidden layer (labelled as 220), the third hidden layer (labelled as 230), the fourth hidden layer (labelled as 240), and an output layer (labelled as 300), wherein the input layer (labelled as 100) comprises three input layer nodes, the first hidden layer (labelled as 210) and the fourth hidden layer (labelled as 240) comprise seven hidden layer nodes, and the second hidden layer (labelled as 220) and the third hidden layer(labelled as 240) comprise four hidden layer nodes, and the output layer (labelled as 300) comprises an output node. In this embodiment, the "Sigmoid" function and the "ReLU" function are used as activation functions respectively.

In this embodiment, the input vector input from the input node is three promoters respectively regulated and controlled by three metals, such as lead and cadmium, and three input genes regulated by the promoter (Fhl -1, Reb -1, Sfp -1, or the like.), and additionally uses 20 genes of known linear relationships as hidden layer genes, such as ALX4, Otx -1, HOXC10, Pknox2, Hoxd13, Dlx1, En2, Prrx2, LHX8, Gbx2, Msx3, Vsx1, Barhl1, Hoxd11, Shox2, Rhox1 1, LRX3, Hoxd10 and Gbx1. The Dlx2 serves as seven hidden layer nodes of the first hidden layer (labelled as 210) and the fourth hidden layer (labelled as 240), and four hidden layer nodes of the second hidden layer (labelled as 220) and the third hidden layer (labelled as 230). In other selected embodiments of the present invention, other genes of known linear relationship can be selected for regulation (referring to "Visual Transcription Factor Dynamics in Moving Cells ". J Cell Biol. 217.4 (2018): 1181-1191, by Liu Zhe, and Robert Tjjian; and "Transcriptional regulatory circuits: predicting numbers from alphabets." Science: 325.5939 (2009): 429-432by Kim Harold D, et al.). In the present invention, the output layer (labelled as 300) includes a green fluorescent protein. In the present example, the toxicity results are predicted by the activity of Agrobacterium cells. The experimental result used as a comparison selects the previously published data (referring to "Assessment of combined toxicity of heavy metals from industrial wastewaters on Photobacterium phosphoreum T3S.", Applied Water Science, 7.4 (2017): 2043-2050 by Zeb B. S.).

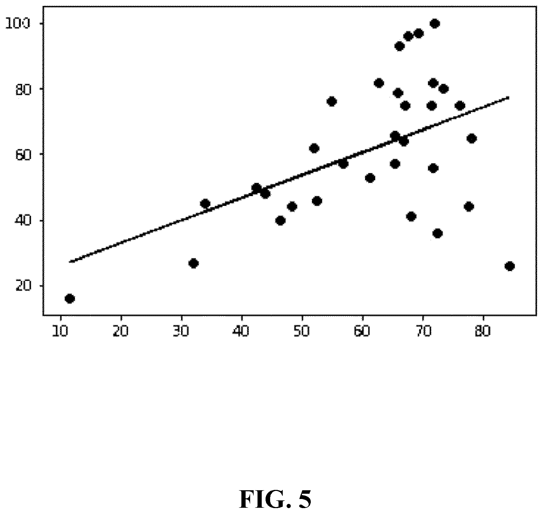

In this embodiment, we return the artificial neural network to the improved result when four hidden layers (seven, four, four, seven hidden layer genes are used as artificial neurons, respectively). The present example compares the activation function of "Sigmoid" (R = - 0.283) and "ReLU" (R = 0.496), and when the "ReLU" is used, the present example can achieve a better result. Through experiments and computer simulation analysis, the gene circuit of the simulated artificial neural network shown in Fig. 4 can effectively predict the toxicity test result (R = 0.496, P < 0.01), reducing the time and money required for testing, and the results thereof refer to Fig. 5, the X-axis is the predicted activity, and the Y-axis is the actual activity; (R = 0.496, P < 0.01).

Therefore, the gene circuit of the simulated artificial neural network according to the present invention can be implemented by using the gene circuit structure to simulate the artificial neural network, so as to simulate complex operations at the molecular level, thereby implementing complex applications in various fields. The gene circuit of the artificial neural network disclosed by the invention enables biological calculation at the intracellular or molecular level to reach the standard required by current mainstream computer science, which is favourable for realizing a rapid and accurate detection of medicine or chemical components. At the same time, it is also beneficial to realize personalized medical treatment and allow diagnosis and medical treatment at the cellular and molecular levels.

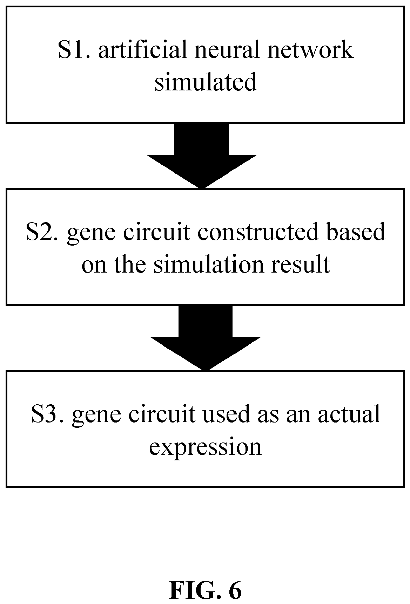

Fig. 6 is a flowchart of a method for constructing a gene circuit of a simulated artificial neural network according to the present invention. As shown in Fig. 6, in step S1, the artificial neural network is simulated. In a selected embodiment of the present invention, the artificial neural network may be any known artificial neural network, such as a BP artificial neural network, a recurrent artificial neural network or the like.

The gene circuit is constructed based on the simulation result shown in step S2. The gene circuit comprises at least one input layer, at least two hidden layers and at least one output layer, wherein the input layer comprises a plurality of input layer nodes, each hidden layer comprises a plurality of hidden layer nodes, the output layer comprises a plurality of output layer nodes. An input vector of each input layer node is a promoter and an input gene, each hidden layer node comprises a hidden layer gene, and each hidden layer gene is controlled by the promoter of that layer or the gene product of the previous layer gene. The output node outputs a gene circuit result; the hidden layer gene and the output layer node are subjected to regulation and control of the gene product of the previous layer of the gene to meet the activation function. The input layer node is subjected to the regulation of the input promoter to meet the activation function. The type and the number of hidden layer genes and the number of hidden layers is based on the gene circuit, according to the present invention. The expert in this field knows how to construct the gene circuit of the simulated artificial neural network shown in any embodiment in Figs. 1-5, thus it will not be discussed here.

In step S3, the gene circuit is used as an actual expression. The expert in this field knows the constructed gene circuit of the simulated artificial neural network would deliver the actual expression as shown in any embodiment in Figs. 1-5, thus it will not be discussed here. After constructing the gene circuit of the simulated artificial neural network of the present invention, the circuit can be applied to numerous different systems such as animal and plant cells, human cells, bacteria, fungi and cell- free systems.

According to the construction method of the gene circuit for simulating the artificial neural network, the artificial neural network can be simulated by adopting the gene circuit structure, so that complex operation can be simulated at the molecular level, in order to realize the complex application in biological fields.

The above only includes the preferred embodiments of the present invention and are not intended to limit the present invention, and any modifications, equivalent substitutions and improvements made within the spirit and principle of the present invention shall fall within the patent protection scope of the present invention.

## Claims

1. A gene circuit for simulating an artificial neural network comprising at least one input layer, at least two hidden layers and at least one output layer, wherein the input layer comprises a plurality of input layer nodes, each hidden layer comprising a plurality of hidden layer nodes, and wherein the output layer comprises a plurality of output layer nodes, wherein an input vector of each input layer node is a promoter and an input gene, wherein each hidden layer node comprises a hidden layer gene, and each hidden layer gene is regulated by a promoter of that layer or a gene product of the previous layer gene, wherein the output layer nodes output the result of the gene circuit, wherein the hidden layer and the output layer nodes are subjected to the regulation and control of the gene product(s) of the previous layer of the gene(s), to meet the activation function; and the input layer nodes are regulated by their promoters to meet the activation function.

2. The gene circuit for simulating an artificial neural network according to claim 1, wherein the gene product is a protein or an RNA.

3. The gene circuit for simulating an artificial neural network according to claim 1 or 2, wherein the output layer for classification analysis or regression analysis.

4. The gene circuit for simulating an artificial neural network according to claim 3, wherein the input layer comprises three input layer nodes, including promoters regulated by ESR 1, PGR, ERBB2, respectively, and these three input genes are regulated by that specific promote, wherein the hidden layer comprises the first hidden layer and the second hidden layer, wherein the first hidden layer comprises six hidden layer genes, and the second hidden layer comprises three hidden layer genes, and these hidden layer genes are comprised of ALX4, OTX1, Hoxc 10, Pknox2, Hoxd13, Dlx1, En2, Prrx2 and Lhx8; and the activation function is a "Sigmoid" function.

5. The gene circuit for simulating an artificial neural network according to claim 4, wherein the classification result of gene circuit on subtype breast cancer: Basal, HER2, and Luminal (ER +).

6. The gene circuit for simulating an artificial neural network according to claim 5, wherein the result of gene circuit on gene synthesis drug PD-L1 for the BASAL subtype breast cancer, a gene synthesis drug NetPipelt-S for the HER2 subtype breast cancer, and a gene synthesis drug Goserelin for the Luminal (ER +) subtype breast cancer.

7. The gene circuit for simulating an artificial neural network according to claim 3, wherein the input layer comprises three input layer nodes, the input nodes each comprises a promoter controlled by copper, lead, cadmium respectively; and three input genes regulated by its specific promoter, wherein the hidden layer comprises the first hidden layer, the second hidden layer, the third hidden layer and the fourth hidden layer, wherein the first hidden layer and the fourth hidden layers both comprise seven hidden layer genes, wherein the second hidden layer and the third hidden layers both comprise four hidden layer genes; and the activation function is a "Sigmoid" function or a "ReLU" function.

8. A method of constructing a gene circuit for simulating an artificial neural network, comprising S1) analog training of Artificial Neural Network and S2) constructing a gene circuit based on a simulation result, wherein the gene circuit comprises at least one input layer, at least two hidden layers and at least one output layer, wherein the input layer comprises a plurality of input layer nodes, each hidden layer comprises a plurality of hidden layer nodes, and the output layer comprises a plurality of output layer nodes, wherein an input vector of each input layer node is a promoter and an input gene, wherein each hidden layer node comprises a hidden layer gene, and each hidden layer gene is regulated by a promoter of that layer or a gene product of the previous layer gene, wherein the output layer nodes output a gene circuit result and the hidden layer and the output layer nodes are subjected to the regulation and control of the gene product(s) of the previous layer of gene(s) to meet the activation function, wherein the number and type of the input gene and hidden genes, and the number of hidden layers are selected based on the gene circuit, wherein the input layer nodes are regulated by their promoters to meet the activation function, wherein the gene circuit simulating artificial neural network mentioned in the present invention is also **characterized by** the gene circuit that is used as an actual expression.

9. The construction method according to claim 8, wherein the input layer that comprises three input layer nodes comprising a promoter regulated by ESR 1, PGR, ERBB2 respectively and three input genes regulated by that specific promoter, wherein the hidden layer comprises the first hidden layer and the second hidden layer, the first hidden layer comprises six hidden layer genes, and the second hidden layer comprises three hidden layer genes, wherein the activation function is a "Sigmoid" function.

10. The construction method according to claim 9, wherein the input layer comprises three input layer nodes which comprise a promoter controlled by heavy metal copper, lead, cadmium respectively, and three input genes regulated by that specific promoter, wherein the hidden layer comprises the first hidden layer, the second hidden layer, the third hidden layer and the fourth hidden layer, wherein the first hidden layer and the fourth hidden layer both comprise seven hidden layer genes, wherein the second hidden layer and the third hidden layer both comprise four hidden layer genes, wherein the activation function is a "Sigmoid" function or a "ReLU" function.
